# EUROPÄISCHE PATENTANMELDUNG

(11) **EP 3 403 689 A1**
(43) Veröffentlichungstag der Anmeldung: **21.11.2018**
(21) Anmeldenummer: 17000828.8
(22) Anmeldetag: 15.05.2017
(51) Int. Cl.: A61N 1/36

(54) **GERÄT ZUR MOBILEN MIKROSTROMANALYSE, MIKROSTROMTHERAPIE, BIOGNOSE**

(71) Anmelder: Herbst, Martin, 5090 Lofer (AT)
(72) Erfinder: Herbst, Martin, 5090 Lofer (AT)

(57) **Zusammenfassung**

MOMICS ist eine Vorrichtung für Mobiltelefone bzw. Mobilgeräte und Datengeräte mit festem oder auch mobilem Betriebssystem, welche Mikrostromanalysen, Mikrostromtherapie sowie Biognose an allen lebenden Organismen durchführt sowie eine zugehörige Anwendungssoftware für den Einsatz auf mobilen Betriebssystemen für Mobiltelefone bzw. Mobilgeräte. Bei der Vorrichtung handelt es sich um eine Erweiterung für Mobiltelefone bzw. Mobil- und Datengeräte aller Art, welche über die Schnittstelle des Mobiltelefons bzw. bzw. des Mobil- und Datengerätes angesteckt wird. Mithilfe der dazugehörigen Anwendungssoftware für den Einsatz auf mobilen Betriebssystemen für Mobiltelefone bzw. Mobilgeräte können die im Körper fließenden Mikroströme, Körpersignale und Informationen gemessen und ausgewertet werden. Kommt bei der Messung zum Vorschein, dass es im Körper ein Problem gibt (etwa das Vorliegen einer bestimmten Krankheit), kann mit MOMICS durch die Abgabe von Frequenzen und Mikrostrom ein Ausgleich des Mikrostromes im Körper herbeigeführt und durch Biognose und Energieren positiv verändert werden, was in weiterer Folge das erkannte Problem therapiert.

## Beschreibung

Die Erfindung bezieht sich auf eine Vorrichtung für Mobiltelefone bzw. Mobilgeräte, welche Mikrostromanalysen und Mikrostromtherapie und die eingetragene und hocherfolgreiche neue Methode der Biognose durchführt sowie eine zugehörige Anwendungssoftware für Mobilgeräte bzw. mobile Betriebssysteme. Bei der Vorrichtung handelt es sich um eine Erweiterung für Mobiltelefone bzw. Datengeräte welche über eine Schnittstelle angesteckt wird oder drahtlos kommuniziert.

Sobald die Vorrichtung an ein Mobiltelefon oder sämtliche andere Kommunikationsgeräte angeschlossen ist, kann mithilfe der mitgelieferten Anwendungssoftware ein Programm zur Körperanalyse mit nachfolgender Mikrostromtherapie gestartet werden. Der Zugang zu dieser Anwendungssoftware erfolgt über ein vom Benutzer zu wählende Zugangsart (Passwort, Fingerprint, Iris-Scan etc.).

Ist die Vorrichtung angesteckt und die Anwendungssoftware gestartet, wird der Benutzer zuerst aufgefordert Elektroden in die entsprechenden Buchsen der Vorrichtung zu führen und diese dann anzulegen. Wie dies zu erfolgen hat, sieht der Benutzer auf dem Bildschirm des Mobiltelefons bzw. Mobilgeräts dargestellt.

Wurden die Elektroden so wie beschrieben angelegt, startet das Programm automatisch bzw. wird der Benutzer aufgefordert, die Start-Taste am Bildschirm zu drücken. Sodann kann der Benutzer der Anzeige sowie den akustischen Signalen und der über den Lautsprecher des Mobiltelefons bzw. Mobilgeräts kommenden Sprachausgabe folgen. Die Sprachausgabe ist in insgesamt über 100 Sprachen verfügbar. An der Vorrichtung selbst befinden sich zusätzliche LED Lampen und Signalgeber.

Anschließend erfolgt im "Biognose Body Check" eine Messung von Signalen und Frequenzen im Rahmen derer die Mikrostromflüsse im Körper analysiert, ausgewertet und die Probleme des Körpers erkannt werden und der Biognoseprozess durchgeführt wird.

In einem weiteren Schritt hat der Benutzer die Möglichkeit, vollautomatisch ein spezielles auf die in seinem Körper erkannten Probleme zugeschnittenes Programm zur Mikrostromtherapie, also zum Ausgleich von jedweder Störung seines Körpers, zu starten, kurzum zu energieren. Optional sind in der Anwendungssoftware aber auch fixe Programme vorgegeben. Der Beginn eines Programmes erfolgt automatisch oder durch Betätigung der Start Taste am Bildschirm des Mobiltelefons bzw. Mobilgeräts. Während der Durchführung der Biognose und Mikrostromtherapie, in Rahmen derer Mikrostrom an den Körper abgegeben wird, erfolgt gleichzeitig die Aufzeichnung und Verarbeitung der veränderten Mikrostromwerte im Körper. Ob diese Daten erfasst und ausgewertet werden, kann der Benutzer bei Start der Anwendungssoftware selbst bestimmen.

Vor jeder weiteren Anwendung kann eine Mikrostromanalyse sowie eine Messung von Signalen und Frequenzen und die Auswertung der bisherigen Daten des Benutzers erfolgen. Diese Daten werden wahlweise direkt am MOMICS oder auf zugewiesenen Servern abgespeichert und auf Wunsch des Benutzers weiterverarbeitet.

Ergibt diese Biognose und Mikrostromanalyse eine Veränderung der Körperwerte, erfährt der Benutzer dies via Bildschirmmitteilung zusammen mit entsprechenden Auswertungen

Die Erfindung wurde im Jahr 2012 entwickelt, im Jahr 2014 weiterentwickelt und erfolgreich getestet.

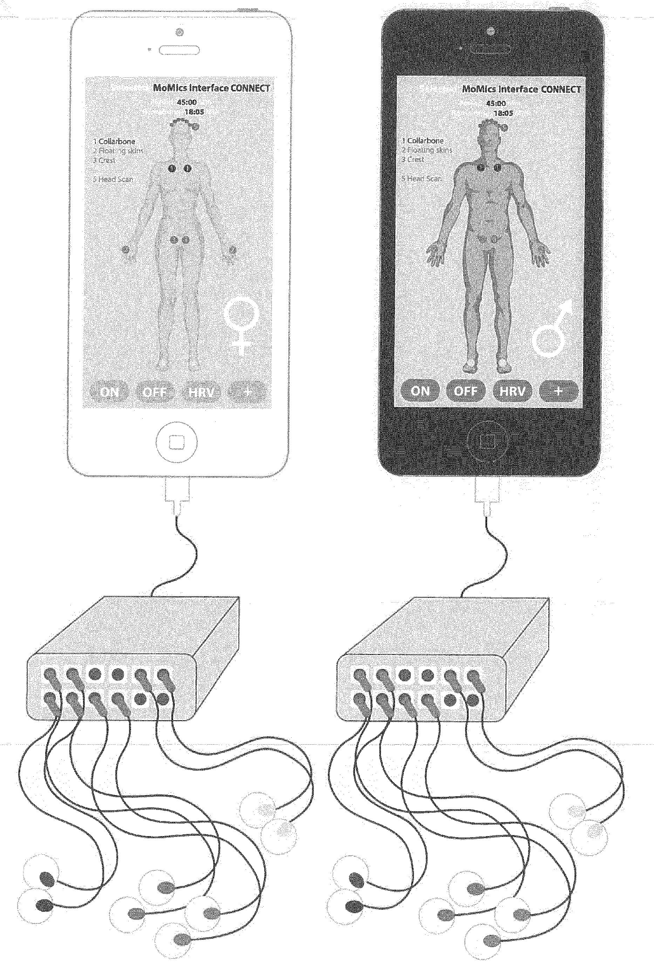

## Patentansprüche

1. **Vorrichtung zum Anschluss an ein Mobiltelefon bzw. Mobilgerät über eine draht- oder drahtlose Schnittstelle, umfassend:** Elektroden zur Messung von Signalen und Frequenzen und zur Mikrostromtherapie und Biognose, wobei mit der Vorrichtung Signalen und Frequenzen aufgezeichnet und ausgewertet und Mikrostrom abgegeben wird.

2. **Anwendungssoftware für den Einsatz auf mobilen und nicht mobilen Betriebssystemen für Mobiltelefone bzw. Mobil- und Datengeräten,** womit die Vorrichtung über das Mobiltelefon bzw. Mobilgerät und Datengeräte sowohl online als auch offline gesteuert werden kann bzw. die Daten sowohl dezentral, als auch direkt auf dem Mobiltelefon bzw. Mobilgerät ausgewertet und gespeichert werden können.

3. **Auslesung, Messung, Steuerung und Therapie mit Mikrostrom sowie Biognose an jedem lebenden Organismus.**
